# EUROPEAN PATENT SPECIFICATION

(11) **EP 0 414 816 B1**
(45) Date of publication and mention of the grant of the patent: **27.07.1994**
(21) Application number: 89907436.3
(22) Date of filing: 18.05.1989
(51) Int. Cl.: A61K 37/00, A61K 39/00, A61K 39/395

(54) **INDUCTION OF TOLERANCE TO A FOREIGN ANTIGEN**
TOLERANZINDUKTION GEGENÜBER EINEM FREMDANTIGEN
INDUCTION DE TOLERANCE A UN ANTIGENE ETRANGER

(30) Priority: 19.05.1988 US 196036
(43) Date of publication of application: 06.03.1991
(73) Proprietor: THE BETH ISRAEL HOSPITAL ASSOCIATION, Boston, MA 02215 (US); BRIGHAM AND WOMEN'S HOSPITAL, Boston, Massachusetts 02115 (US)
(72) Inventor: STROM, Terry, B., Brookline, MA 02146 (US); KELLEY, Vicki, E., Brookline, MA 02146 (US)
(74) Representative: Deans, Michael John Percy
(86) International application number: US8902166
(87) International publication number: WO8911287

(56) References cited:
- EP-A- 0 240 344
- US-A- 4 545 985
- US-A- 4 675 382
- US-A- 4 681 760
- PROC. NATL. ACAD. SCI. USA, vol,. 85, March 1988, pages 1922-1926; H.Lorberboum-Galski et al.
- PROTEIN ENGINEERING, vol. 1, no. 6, 1987, pages 493-498; D.P. WILLIAMS et al.
- CHEMICAL ABSTRACTS, vol. 78, no. 13, 2nd April 1973, pages 315-316, abstract no. 82863f, Columbus, Ohio, US; J.M. Fidler et al.

## Description

This invention relates to the prevention of unwanted immune responses to foreign antigens.

Foreign antigens (e.g. recombinant proteins) administered to mammals, e.g. humans, for therapeutic purposes can cause unwanted immune responses, i.e. the formation of antibodies against the foreign antigen. Such immune responses can occur even where the antigen is the recombinant form of a naturally-occurring human protein, e.g. tissue plasminogen activator, because such a protein, even if having the same amino acid sequence as the natural protein, can be glycosylated in such a way as to cause the protein to appear foreign to the immune system. Thus, the term "foreign antigen" as used herein refers to any substance which is not identical to a substance naturally present in the mammal being treated. A goal of medicine is to be able to administer foreign therapeutic agents such as recombinant proteins, without evoking an unwanted immune response, i.e. to induce tolerance to the therapeutic agent. (Tolerance as used herein means the suppression of the ability to amount a humoral immune response to a foreign antigen upon re-challenge with that antigen, even after the substance inducing the suppression has been cleared from the bloodstream).

In a first aspect of the invention we provide a hybrid protein comprising a first and a second proteinaceous portion joined together covalently, said first portion comprising the enzymatically active portion of a toxin molecule, and said second portion comprising a portion of interleukin-2 (hereafter "IL-2") or an IL-2 receptor-binding fragment or an analog of either thereof and a foreign antigen for concomitant use (i.e. simultaneous administration to an animal or human patient or administration of said hybrid protein after said foreign antigen to a human or animal patient, preferably within 7 days of supply of the foreign antigen) in therapy or for prophylaxis.

In a second aspect of the invention we provide a hybrid protein comprising a first and a second proteinaceous portion joined together covalently, said first portion comprising the enzymatically active portion of a toxin molecule, and said second portion comprising a portion of interleukin-2 (hereafter "IL-2") or an IL-2 receptor-binding fragment or an analog of either thereof; and a foreign antigen for concomitant use (i.e. simultaneous administration to an animal or human patient or administration of said hybrid protein after said foreign antigen to a human or animal patient, preferably within 7 days of supply of said foreign antigen), whereby to suppress said animal or human patient's humoral immune response to a subsequent challenge with said antigen by destruction to a sufficient degree of IL-2-receptor-positive-cells, preferably helper T-cells. The toxin molecule preferably is a bacterial toxin, most preferably, diphtheria toxin or alternatively Pseudomonas exotoxin.

Preferably tolerance is permanent; re-challenge with the foreign antigen at any time, even after the substance has cleared from the mammal's bloodstream, results in a suppression of the mammal's ability to mount a humoral immune response to the antigen. The antigen-specific induced state of tolerance achieved according to the invention can greatly improve disease-related therapy, e.g. short-term or long-term therapy involving administration of a monoclonal antibody, a recombinant protein, or other medically useful foreign antigens.

### Description of the Preferred Embodiments

Tolerance to the foreign antigen trinitrobenzenesulfonic acid (TNBS) was defined as the inability of a mammal, after being re-challenged with a normally immune response generating (i.e., antibody-stimulating) dose of TNBS thirty days following administration of both TNBS and the tolerance inducing substance, to mount a humoral response to the re-challenge, as demonstrated by the absence in the mammal's bloodstream of circulating anti-TNBS antibodies following re-challenge.

Tolerance was achieved and demonstrated generally according to the following steps: 1) obtaining the tolerance-inducing substance; 2) immunization of the test mammals (mice) with TNBS; 3) administration of the tolerance-inducing substance to the test mammals; 4) re-challenge of the test mammals with TNBS; 5) determination of the normal response of tolerance-inducing-substance treated test mammals to a variety of antigens administered to the animals asynchroniously with tolerance inducing substance; and 6) characterization of the immune response or lack thereof, following re-challenge, by quantitation of anti-TNBS antibodies and characterization of T-lymphocyte surface markers.

### Preparation of Chimeric IL-2-toxin Fusion Protein

One embodiment of the invention employs, as the IL-2-receptor-positive-cell-destroying substance, a chimeric IL-2-receptor-specific toxin. Expression of the IL-2-receptor is a necessary, albeit transient, step in the common pathway of T-cell activation, and thus the IL-2 receptor is found on newly-activated helper T-cells, but not on older activated T-cells and resting T-cells.

The 68,086 dalton fusion protein, IL-2-toxin, was expressed from a genetically constructed hybrid gene encoding both a portion of diphtheria toxin and IL-2, in which DNA coding for the diphtheria toxin's generalized eukaryotic receptor binding domain was replaced with IL-2-encoding DNA, using recombinant DNA methods, as described in Murphy U.S. Patent No. 4,675,382. The genetic construction was carried out as follows, under the direction of John R. Murphy, University Hospital, Boston, MA.

The diphtheria toxin-related portion of the fusion gene was carried on plasmid pABC508 (Bishai et al, 1987, J. Bacteriol. 169:1554). This plasmid carries the genetic information encoding the diphtheria tox promoter and the tox structural gene through amino acid Ala₄₈₅. The region of the tox gene encoding Val₄₈₃-His₄₈₄-Ala₄₈₅ is also defined by a unique SphI restriction endonuclease site. The IL-2 portion of the fusion gene was synthesized in vitro and cloned in the pUC18 vector (pUC18 can be obtained from Bethesda Research Labs, Bethesda, MD). The sequence of the IL-2 gene is well known and is given in Taniguchi at al. U.S. Patent No. 4,738,927, hereby incorporated by reference. By design, the 5'-end of the synthetic IL-2 gene was defined by an SphI restriction site on plasmid pDW15 in order to facilitate construction of the toxin/growth factor fusion gene. The chimeric diphtheria toxin-related IL-2 fusion gene was constructed by digestion of pDW15 with SphI and SalI, purification of the 428-bp synthetic IL-2 gene by agarose gel electrophoresus, and recloning of the fragment into SphI and SalI-digested pABC508. The SphI restriction site on the 5'-end of the synthetic IL-2 gene was positioned such that the translational reading frame would be retained through the diphtheria toxin/IL-2 fusion junction, such that Pro₂ of the mature form of IL-2 would be joined to Ala₄₈₅ of diphtheria toxin through a peptide bond.

Following ligation and transformation, several clones of E. coli were isolated that contained plasmids which had the restriction endonuclease digestion patterns expected for the toxin-growth factor gene fusion. One of these strains was selected and the recombinant plasmid designated pABI508. In order to ensure that the correct translational reading frame was maintained for the IL-2 portion of the gene fusion following assembly of the intact chimeric toxin gene, the nucleotide sequence of the IL-2 portion of the chimera through the fusion junction into the toxin portion of the recombinant gene was determined. The DNA sequence and the deduced amino acid sequence in the region of the toxin/T-cell growth factor fusion junction demonstrated that the IL-2 reading frame was retained.

### Expression and Partial Purification of IL-2-toxin

Cloned diphtheria tox gene products possessing a functional signal sequence are expressed and exported to the periplasmic compartment of E. coli K-12. E. coli (pABI508) was grown in 10-L volumes and periplasmic extracts were prepared and analyzed by polyacrylamide gel electrophoresis and immunoblotting. A single protein of Mᵣ 68,000 was found to be immunoreactive with monoclonal antibodies to recombinant IL-2 (rIL-2).

Anti-IL-2 was used as an immunoaffinity matrix for the purification of IL-2 toxin. Concentrated periplasmic extracts from E. coli (pABI508) were applied to an anti-IL-2 column, the column was exhaustively washed, and IL-2-toxin was eluted with 4M guanidine hydrochloride. Typically, immunoaffinity chromatography results in preparations of IL-2-toxin which are 50-70% pure as measured by laser densitometry of SDS-polacrylamide gels which have been Coomassie Blue stained.

### Induction of Tolerance Using IL-2-toxin

Animals BALB/c mice were obtained from the Jackson Laboratory, Bar Harbor, ME, and then maintained in the animal facilities at Brigham and Women's Hospital, Boston, MA. All mice studied were males between 5 and 10 wks of age.

Immunization of Mice with Hapten Mice were immunized with a 10 mm solution of TNBS in 0.5 M phosphate-buffered saline (PBS) at pH 7.2 to 7.4 administered by subcutaneous injection of 0.1 ml bilaterally in the dorsum. 7 days after immunization, these mice were challenge with 25 µl of the same solution into the right footpad. 30 days after immunization, these animals were re-challenged with 0.1 ml of the same solution administered by subcutaneous injection bilaterally into the dorsum.

### Administration of IL-2-Toxin

IL-2-toxin is selectively cytotoxic for both murine and human T cells bearing high affinity surface IL-2 receptors, whereas cells which do not express such receptors are resistant to IL-2-toxin action. Mice were injected intraperitoneally (IP) at daily intervals from the time of immunization through day 7 with a single dose of IL-2-toxin (5 µg per animal) or CRM45, a control substance (5 µg per animal) consisting of a 45,000 dalton molecular weight non-toxic mutant form of diphtheria toxin which lacks the toxin's normal receptor binding domain.

### Characterization of IL-2-Toxin Induced Tolerance

In order to characterize the action of IL-2-toxin induced tolerance, two assays were performed: first, the mono-dispersed lymph node cells of the limb ipsilateral to antigen challenged footpad were phenotyped with T-cell subset and p55 IL-2R markers, using M7/20 monoclonal antibody to detect the p55 IL-2 receptor cell surface marker; and, second, circulating anti-TNBS antibodies were measured using an ELISA assay.

Two monoclonal antibodies were used for detecting T-cell surface markers, the M7/20 monoclonal antibody that is specific for the p55 subunit of the IL-2 receptor, and a control monoclonal antibody, RA3-2C2, that is specific for pre-B cells and some resting B cells, and therefore will not detect the newly activated IL-2 receptor positive cells. These monoclonal antibodies were prepared according to the following method.

### Preparation and Administration of Monoclonal Antibodies

Production and initial screening of monoclonal antibodies to yield those specific for the IL-2 receptor can be carried out as described in Uchiyama et al., 1981, J. Immunol. 126:1393. This method, briefly, is as follows.

Human cultured T-lymphocytes are injected into mammals, e.g., mice, the spleens of the immunized animals are removed, and the spleen cells separated and then fused with immortal cells, e.g., mouse or human myeloma cells, to form hybridomas. The hybridoma culture supernatants are then screened for those that contain antibodies specific for the IL-2 receptor, using a complement-dependent cytotoxicity test. Human T-lymphocytes and EBV transformed B-lymphocytes are labeled with ⁵¹Cr sodium chromate and used as target cells. When the target cells are incubated with antibody-containing culture supernatants in the presence of complement, only those cells that are bound by antibody will lyse and release ⁵¹Cr. The supernatants are collected and the amount of ⁵¹Cr present is determined using a gamma counter. Those supernatants exhibiting toxicity against newly activated (i.e., IL-2 receptor bearing) T-lymphocytes, but not older activated T- or B-lymphocytes, are selected, and then subjected to a further screening step to select those supernatants that contain antibody that is specifically reactive with the IL-2 receptor; such antibody will immunoprecipitate the 50 kilodalton glycoprotein IL-2 receptor (described in detail in Leonard et al., 1983, P.N.A.S. USA 80:6957). The desired anti-IL-2 receptor antibody is purified from the supernatants using conventional methods.

The monoclonal antibody employed was antibody M7/20, which is described in Gaulton et al. (1985) Clin. Immunol. and Immunopath 36:18. M7/20 is a monoclonal rat anti-mouse K, mu, Ig antibody specific for the IL-2 receptor. M7/20 was purified from the culture supernatants of cells grown in serum free media (Hanna Labs, Berkeley, CA). Supernatants were precipitated with 40-50% saturated ammonium sulfate, dialyzed, passed over DEAE Affi-Gel Blue (Bio-Rad, Richmond, VA) in 20 mM NaCl, and the eluate fractionated on Sephadex G-200 (Pharmacia, Piscataway, NJ), run in 20 mM Tris (pH 7.2), 250 mM NaCl, 0.5% n-butanol. Antibody purity was assessed by SDS-Page gel electrophoresis. The control monoclonal antibody, RA3-2C2, was purified from cells obtained from the American Type Culture Collection (Rockville, MD) by the procedure described above.

Phenotyping of Lymph Node Cells Single cell (10⁶ cells/sample) suspensions of draining paraortic lymph nodes of the 7 day TNBS-challenged lymph or spleen cells from IL-2-toxin-treated and control mice were stained with a saturating amount of either anti-L3T4 (prepared from the hybridoma GK1.5, American Type Culture Collection Accession No. T1B207, Rockville, MD.), a rat IgG2b Mab, or anti-Lyt-2 (from the hybridoma 53-6.72, A.T.C.C. Accession No. T1B195), a rat IgG2a, in 50 ul of PBS at pH 7.4 containing 20% heat-inactivated mouse serum (Cappel Laboratories, West Chester, PA), together with 0.1% sodium azide (PBS-S). The cells were counterstained with a fluorescein labeled rabbit anti-rat IgG (Cappel Laboratories) in 50 ul of PBS-S. Biotinylated purified M7/20 was then added to the cell preparation (50 ul) in PBS-S and counterstained with phycoerythrin-avidin in PBS-S (Becton-Dickinson, Mountain View, CA). All incubations were performed on ice for 30 min, and the cells were washed 3x in cold PBS and kept at 4°C in the dark until analysis. The cells were analyzed on a FACS-1 cell analyzer (Becton-Dickinson FACS Systems, Mountainview, CA) using a Consort 30 computer program supplied by Becton-Dickinson. Background staining was determined by incubating cells with FITC rabbit anti-rat Ig followed by biotin labeled sheep anti-rat Ig and phycoerythrin-avidin

IL-2-toxin selectively targeted and eliminated IL-2 receptor bearing T cells in draining lymph nodes. As determined by dual beam flow cytometric analysis the percent of CD4⁺ p55⁺ IL-2R⁺ cells was reduced from 14% in immunized, untreated mice to 5% in TNBS-immunized IL-2-toxin treated animals by day 7. Similarly, the CD8⁺ p55⁺ IL-2R⁺ cells were depleted from 18% to 5% by day 7. Indeed, IL-2-toxin was so effective that it reduced the number of p55⁺ IL-2R⁺ T cells to values similar to the levels detected in non-immunized mice (3% for CD4⁺and 2% for CD8⁺ cells). By contrast, essentially no increase was detected in IL-2 receptor expression in spleen cells after TNBS immunization in IL-2 toxin treated mice, and the low percentage of IL-2R⁺ cells was not significantly different from non-immunized animals.

### Quantitation of Circulating Antibodies

In order to measure the extent to which IL-2-toxin treatment induced a state of tolerance with respect to humoral immunity, circulating antigen-specific antibodies were quantitated from serum obtained from mice on days 0, 7, and 30. The titer of anti-TNBS immunoglobulins present in the serum of IL-2 toxin-treated, TNBS-immunized mice was measured by a solid phase enzyme-linked immunoassay (ELISA). Polystyrene 96-well microtiter plates (COSTAR, Cambridge, MA) were coated with 40 ng of purified TNBS (50 ul/well) in a borate buffer (0.05M, pH 8.6) overnight at 4°C. Plates were then incubated with 3% bovine serum albumin (BSA) in PBS for 1 hr at 25°C. Four fold dilutions of mouse serum ranging from 1:50 to 1:12,800 suspended in 1% BSA, 0.05% Tween-80, PBS (diluting buffer) (50ul) were then added to each well for 4 hrs at 25°C. The wells were then washed with (100 ul) PBS 1% Tween-80xl and PBSx2. Bound serum IgG was detected by incubating the wells with Protein A-alkaline phosphatase conjugate (ZYMED) (1:3,000 dil.) in diluting buffer overnight at 4°C. After the plates were washed, bound alkaline phosphatase conjugate was detected by the addition of 1 mg/ml p-nitrophenyl phosphate (Sigma). The optical density (OD) was read at a wavelength of 405 nm. in a DYNASCAN miltichannel ELISA reader. All tests were performed in triplicate. The end titer was quantitated as the greatest dilution of test mouse serum which possessed significant binding to TNBS in excess of preimmune serum.

IL-2-toxin treatment induced tolerance to the TNBS antigen in TNBS immunized animals. Thirty days after immunization, exposure to TNBS did not stimulate an immune response to this antigen; there were no detectable levels of circulating anti-TNBS antibodies in the IL-2-toxin treated group.

### Mechanism of Tolerance

The mechanism underlying the loss of the ability to respond to a foreign antigen, i.e., the induction of tolerance, is unknown, but may be a deletion of the antigen-specific clone of helper T-cells. The permanence of this clonal deletion may be the result of an attempt by the immune system to replenish this antigen-specific T-cell clone from the stem cell population of the bone marrow, resulting in a new antigen-specific clone of suppressor T-cells rather than helper T-cells.

### Human Dosage and Administration

Dosages of tolerance-inducing substances will vary, depending on factors such as half-life of the substance, potency, and route of administration, and the condition of the patient. Generally, IL-2-toxin should be administered to the patient in such a way that it is present in the vicinty of the foreign antigen at a concentration of about 10⁻⁹ M IL-2-toxin, throughout the first 45 minutes of treatment with the foreign antigen. For example, where the foreign antigen is to be introduced into an adult patient's bloodstream, simultaneous intravenous infusion of a solution of approximately 15 mg IL-2-toxin in an appropriate volume of saline, delivered over the course of one hour, would generally provide an adequate concentration of IL-2-toxin in the blood for an adequate length of time. This protocol may be adjusted to provide for a lower concentration of IL-2-toxin for a longer period, or a higher concentration for a shorter period of treatment. Where the presence of the foreign antigen will be localized rather than systemic, the IL-2-toxin treatment protocol may be appropriately adjusted to provide for 10⁻⁹ M IL-2-toxin in the immediate vicinity of the foreign antigen, for 45 minutes.

### Other Embodiments

Other embodiments of the invention are within the following claims. For example, the Pseudomonas aeruginosa exotoxin may be fused to the IL-2 receptor specific fragment in place of the diphtheria toxin portion of the hybrid protein. The Pseudomonas exotoxin is a well-known toxin, described in Pastan et al., U. S. Patent No. 4,545,985. The gene encoding this toxin is described in Hwang et al., (1987) Cell 48:129.

## Claims

1. A hybrid protein comprising a first and a second proteinaceous portion joined together covalently, said first portion comprising the enzymatically active portion of a toxin molecule, and said second portion comprising a portion of interleukin-2 (hereafter "IL-2") or an IL-2 receptor-binding fragment or an analog of either thereof and a foreign antigen for concomitant use (i.e. simultaneous administration to an animal or human patient or administration of said hybrid protein after said foreign antigen to a human or animal patient, preferably within 7 days of supply of the foreign antigen) in therapy or for prophylaxis.

2. A hybrid protein comprising a first and a second proteinaceous portion joined together covalently, said first portion comprising the enzymatically active portion of a toxin molecule, and said second portion comprising a portion of interleukin-2 (hereafter "IL-2") or an IL-2 receptor-binding fragment or an analog of either thereof and a foreign antigen for concomitant use (i.e. simultaneous administration to an animal or human patient or administration of said hybrid protein after said foreign antigen to a human or animal patient, preferably within 7 days of supply of said foreign antigen, whereby to suppress said animal or human patient's humoral immune response to a subsequent challenge with said antigen by destruction to a sufficient degree of IL-2-receptor-positive-cells, preferably helper T-cells.

3. A hybrid protein and a foreign antigen for concomitant use as defined in Claim 1, further characterised in that said first portion and said second portion are joined together by a peptide bond.

4. A hybrid protein and a foreign antigen for concomitant use as defined in any of Claims 1 to 3, further characterised in that said toxin molecule comprises a bacterial toxin, preferably diphtheria toxin or Pseudomonas exotoxin.

5. Use of a substance for the production of an infusion for concomitant supply of said substance together with a foreign antigen to an animal or human patient to induce tolerance of said animal or human patient to said foreign antigen, said substance comprising a said hybrid protein as defined in any of Claims 1 to 4.

6. Use of a substance for the manufacture of a medicament for supply to a human or animal patient concomitantly with supply to that same patient of a foreign antigen in order to induce tolerance of said patient to said foreign antigen, said substance comprising a said hybrid protein as defined in any of Claims 1 to 4.

## Patentansprüche

1. Hybridprotein, umfassend einen ersten und zweiten kovalent miteinander verbundenen Proteinanteil, wobei der genannte erste Anteil den enzymatisch aktiven Anteil eines Toxinmoleküls und der genannte zweite Anteil einen Anteil von Interleukin-2 (nachstehend "IL-2") oder ein IL-2 rezeptorbindendes Fragment oder ein Analog irgendeines dieser beiden umfaßt, sowie ein Fremdantigen zur begleitenden Anwendung (d.h. zur gleichzeitigen Verabreichung an einen tierischen oder menschlichen Patienten oder zur Verabreichung des genannten Hybridproteins nach dem genannten Fremdantigen an einen tierischen oder menschlichen Patienten, vorzugsweise innerhalb von 7 Tagen ab Verabreichung des Fremdantigens) zur Therapie oder Prophylaxe.

2. Hybridprotein, umfassend einen ersten und zweiten kovalent miteinander verbundenen Proteinanteil, wobei der genannte erste Anteil den enzymatisch aktiven Anteil eines Toxinmoleküls und der genannte zweite Anteil einen Anteil von Interleukin-2 (nachstehend "IL-2") oder ein IL-2 rezeptorbindendes Fragment oder ein Analog irgendeines dieser beiden umfaßt, sowie ein Fremdantigen zur begleitenden Anwendung (d.h. zur gleichzeitigen Verabreichung an einen menschlichen oder tierischen Patienten oder zur Verabreichung des genannten Hybridproteins nach dem genannten Fremdantigen an einen menschlichen oder tierischen Patienten, vorzugsweise innerhalb von 7 Tagen ab Verabreichung des Fremdantigens), wodurch die humorale Immunreaktion des genannten menschlichen oder tierischen Patienten auf eine nachfolgende Herausforderung durch dieses Antigen durch Zerstörung eines ausreichenden Ausmaßes von IL-2-rezeptorpositiven Zellen, vorzugsweise Helfer T-Zellen, unterdrückt wird.

3. Hybridprotein und ein Fremdantigen zur begleitenden Anwendung nach Anspruch 1, weiters dadurch gekennzeichnet, daß der genannte erste und der genannte zweite Anteil durch eine Peptidbindung miteinander verbunden sind.

4. Hybridprotein und Fremdantigen zur begleitenden Anwendung nach einem der Ansprüche 1 bis 3, weiters dadurch gekennzeichnet, daß das genannte Toxinmolekül ein bakterielles Toxin, vorzugsweise Diphtherietoxin oder Pseudomonas-Exotoxin umfaßt.

5. Verwendung einer Substanz zur Herstellung einer Infusion zur begleitenden Verabreichung der genannten Substanz gemeinsam mit einem Fremdantigen an einen tierischen oder menschlichen Patienten, um eine Verträglichkeit des genannten tierischen oder menschlichen Patienten gegenüber dem genannten Fremdantigen hervorzurufen, wobei die genannte Substanz ein Hybridprotein nach einem der Ansprüche 1 bis 4 umfaßt.

6. Verwendung einer Substanz zur Herstellung eines Medikaments zur Verabreichung an einen menschlichen oder tierischen Patienten unter begleitender Verabreichung eines Fremdantigens an denselben Patienten, um eine Verträglichkeit des genannten Patienten gegenüber dem genannten Fremdantigen hervorzurufen, wobei die genannte Substanz ein Hybridprotein nach einem der Ansprüche 1 bis 4 umfaßt.

## Revendications

1. Protéine hybride comprenant une première et une seconde partie protéiques réunies ensemble par covalence, ladite première partie comprenant la partie enzymatiquement active d'une molécule de toxine et ladite seconde partie comprenant une partie d'interleukine-2 (notée ci-après "IL-2") ou un fragment ligand-récepteur de IL-2 ou une partie analogue à l'un ou l'autre et un antigène étranger en vue d'une utilisation concomitante (c'est-à-dire l'administration simultanée à un animal ou à un être humain, ou l'administration de ladite protéine hybride après ledit antigène étranger à un être humain ou à un animal, de préférence dans les 7 jours suivant l'administration de l'antigène étranger) en thérapie ou à des fins de prophylaxie.

2. Protéine hybride comprenant une première et une seconde partie protéiques réunies ensemble par covalence, ladite première partie comprenant la partie enzymatiquement active d'une molécule de toxine, et ladite seconde partie comprenant une partie d'interleukine-2 (notée ci-après "IL-2") ou un fragment ligand-récepteur de IL-2 ou une partie analogue à l'un ou l'autre et un antigène étranger en vue d'une utilisation concomitante (c'est-à-dire l'administration simultanée à un animal ou à un être humain, ou l'administration de ladite protéine hybride après ledit antigène étranger à un être humain ou à un animal, de préférence dans les 7 jours suivant l'administration dudit antigène étranger), pour supprimer de ce fait la réponse immunitaire humorale dudit animal ou être humain à une compétition ultérieure avec ledit antigène par destruction à un degré suffisant des cellules de récepteur positif de IL-2, de préférence des cellules T auxiliaires.

3. Protéine hybride et antigène étranger en vue d'une utilisation concomitante ainsi que définis dans la revendication 1, caractérisés en outre par le fait que ladite première partie et ladite seconde partie sont réunies ensemble au moyen d'une liaison peptidique.

4. Protéine hybride et antigène étranger en vue d'une utilisation concomitante selon l'une quelconque des revendications 1 à 3, caractérisés en outre par le fait que ladite molécule de toxine comprend une toxine bactérienne, de préférence une toxine diphtérique ou une exotoxine Pseudomonas.

5. Utilisation d'une substance pour la production d'une infusion en vue d'une administration concomitante de ladite substance avec un antigène étranger à un animal ou un être humain pour induire une tolérance de la part dudit animal ou être humain vis-à-vis dudit antigène étranger, ladite substance comprenant une telle protéine hybride ainsi qu'il est défini dans l'une quelconque des revendications 1 à 4.

6. Utilisation d'une substance pour la fabrication d'un médicament en vue de l'administration à un être humain ou à un animal en concomitance avec l'administation au même patient d'un antigène étranger afin d'induire une tolérance de la part dudit patient audit antigène étranger, ladite substance comprenant une telle protéine hybride ainsi qu'il est défini dans l'une quelconque des revendications 1 à 4.
